# EUROPEAN PATENT APPLICATION

(11) **EP 4 678 179 A1**
(43) Date of publication of application: **14.01.2026**
(21) Application number: 23926242.1
(22) Date of filing: 07.03.2023
(51) Int. Cl.: A61K 38/05, A61K 31/198, A61K 38/08, A61P 1/00, A61P 11/00, A61P 11/06

(54) **PHARMACEUTICAL COMPOSITION CONTAINING ACTIVE SULFUR MOLECULE**

(71) Applicant: Tohoku University, Sendai-shi, Miyagi 980-8577 (JP)
(72) Inventor: ISHII Naoto, Sendai-shi, Miyagi 980-8577 (JP); NUMAKURA Tadahisa, Sendai-shi, Miyagi 980-8577 (JP); YAMADA Mitsuhiro, Sendai-shi, Miyagi 980-8577 (JP); SUGIURA Hisatoshi, Sendai-shi, Miyagi 980-8577 (JP); AKAIKE Takaaki, Sendai-shi, Miyagi 980-8577 (JP)
(74) Representative: Hoffmann Eitle
(86) International application number: PCT/JP2023/008528
(87) International publication number: WO 2024/185028

(57) **Abstract**

The present invention relates to a pharmaceutical composition for preventing or treating an inflammatory bowel disease, a type 2 airway inflammation or the like, containing an active sulfur molecule as an active ingredient. Specifically, the present invention relates to a pharmaceutical composition for treating or preventing an inflammatory bowel disease or a type 2 airway inflammation, containing a persulfide represented by Formula (1) described below or a pharmaceutically acceptable salt or solvate thereof: R¹S(S)ₙ R² (1), where R¹ and R² are independently selected from L-cysteine, homocysteine, and glutathione (GSH), and represent moieties other than a thiol group; and n is an integer of 2 or more.

## Description

### Technical Field

The present invention relates to a pharmaceutical composition for preventing or treating an inflammatory bowel disease, a type 2 airway inflammation or the like, containing an active sulfur molecule as an active ingredient.

### Background Art

Inflammatory bowel diseases (IBDs) are diseases characterized by chronic digestive tract inflammation, in some of which abnormal responses of CD4⁺ T cells are observed. Recent studies have revealed that abnormal responses of T cells in autoimmune diseases and IBDs may be associated with disruption of the balance between glycolysis and mitochondrial metabolism in the same cells.

The inventors have reported that CysSSH, which is produced by cysteine tRNA synthetase (CARS2) involved in sulfur metabolism in mitochondria, may suppress proliferation of pathogenic CD4+ T cells during intestinal inflammation (Non-Patent Document 1).

The inventors have also reported that in type 2 airway inflammation models created by transnasal administration of a mite antigen to mice heterozygously deficient in CARS2, an increase in proportion of cells positive for type 2 cytokines and GATA3 was observed, indicating that an active sulfur molecule (RSS) may be involved in the regulation of a type 2 airway inflammation (Non-Patent Document 2).

Active sulfur molecules are known to play an important role in antioxidative stress responses in vivo (Non-Patent Document 3). Among the active sulfur molecules, a persulfide having a plurality of sulfur molecules such as glutathione trisulfide (GSSSG) has a stronger antioxidative effect. It has been reported that the active sulfur molecules have anti-inflammatory effect, and inhibit inflammations mediated by TLR4, inhibit activation of NF-κB mediated by IL-1β and IL-6, and inhibit activation of NLRP3 inflammasome.

There have been reported an intraocular irrigating solution (Patent Document 1), an antiviral drug, an immunomodulator (for a viral infection), and an anti-inflammatory agent (Patent Document 2) each containing an active sulfur molecule, which utilize the antioxidative and anti-inflammatory effects of the active sulfur molecule. These patent documents indicate the anti-inflammatory and antiviral effects of the active sulfur molecules, but do not indicate their immunosuppressive functions.

### Citation List

### Patent Document

Patent Document 1: WO2017/005768
Patent Document 2: WO2021/235494

### Non-Patent Document

Non-Patent Document 1: Tayama et al., Reactive sulfide species generated by cysteinyl-tRNA synthetase plays a regulatory role in T cell-induced colitis in a T cell-intrinsic manner., The 50th Annual Meeting of the Japanese Society for Immunology, Meeting Abstracts, Dec. 9, 2021
Non-Patent Document 2: Sasaki et al. Deficiency of CARS2, a principal cysteine persulfide synthase, aggravated house dust mite-induced allergic inflammation in a mouse model of asthma. European Respiratory Journal 2020 56:310; DOI: 10.1183/13993003.congress-2020.310
Non-Patent Document 3: Ida, T. et al., Reactive cysteine persulfides and S-polythiolation regulate oxidative stress and redox signaling. Proc. Natl. Acad. Sci. USA,2014, doi: 10.1073/pnas.1321232111

### Summary of Invention

### Technical Problem

An object of the present invention is to elucidate the role of an active sulfur molecule in a T cell immune response and to provide a new application of the active sulfur molecule in clinical practice.

### Solution to Problem

The inventors have found that administration of glutathione persulfides improves pathological conditions in models of inflammatory bowel diseases and type 2 airway inflammations, and that the effect is attributed to the regulation of CD4⁺ T cells by the glutathione persulfides.

The present invention is based on the above findings and relates to the following [1] to [9].
[1] A pharmaceutical composition for treating or preventing an inflammatory bowel disease or a type 2 airway inflammation, the pharmaceutical composition containing a persulfide represented by Formula (1) described below or a pharmaceutically acceptable salt or solvate thereof:

   R¹S(S)ₙR² (1),

   where R¹ and R² are independently selected from L-cysteine, N-acetylcysteine, homocysteine, and glutathione (GSH), and represent moieties other than a thiol group; and n is an integer of 2 or more.
[2] The pharmaceutical composition according to [1], in which R¹ and R² are moieties other than a thiol group of glutathione.
[3] The pharmaceutical composition according to [1] or [2], in which the persulfide is glutathione trisulfide or glutathione tetrasulfide, preferably glutathione trisulfide.
[4] The pharmaceutical composition according to any one of [1] to [3], in which the inflammatory bowel disease is Crohn's disease or ulcerative colitis.
[5] The pharmaceutical composition according to any one of [1] to [4], in which the type 2 airway inflammation is asthma.
[6] The pharmaceutical composition according to any one of [1] to [5], in which the persulfide treats or prevents the inflammatory bowel disease or the type 2 airway inflammation by regulating activation or proliferation of CD4⁺ T cells.
[7] A CD4⁺ T cell modulator containing, as an active ingredient, a persulfide represented by Formula (1) described below or a pharmaceutically acceptable salt or solvate thereof:

   R¹S(S)ₙR² (1),

   where R¹ and R² are independently selected from L-cysteine, N-acetylcysteine, homocysteine, and glutathione (GSH), and represent moieties other than a thiol group; and n is an integer of 2 or more.
[8] The CD4⁺ T cell modulator according to [7], in which R¹ and R² are moieties other than a thiol group of glutathione.
[9] The CD4⁺ T cell modulator according to [7] or [8], in which the persulfide is glutathione trisulfide or glutathione tetrasulfide, preferably glutathione trisulfide.

### Advantageous Effects of Invention

According to the present invention, a novel method for treating diseases associated with abnormalities of CD4⁺ T cells, such as inflammatory bowel diseases and type 2 airway inflammations, is provided.

### Brief Description of Drawings

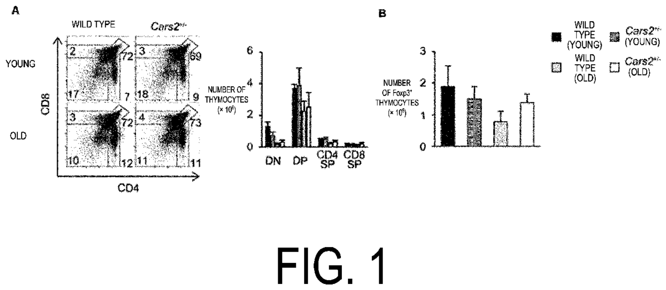

FIG. 1 shows T cell differentiation in the thymi of Cars2+/- mice. (A) Dot plots show the expressions of CD4 and CD8 in thymocytes, and a graph shows the numbers of cells (mean ± SEM) of DN, DP, and CD4/CD8 SP cells (n = from 3 to 8). (B) Absolute numbers of Foxp3+ thymocytes (mean ± standard deviation) are shown (n = from 3 to 8).

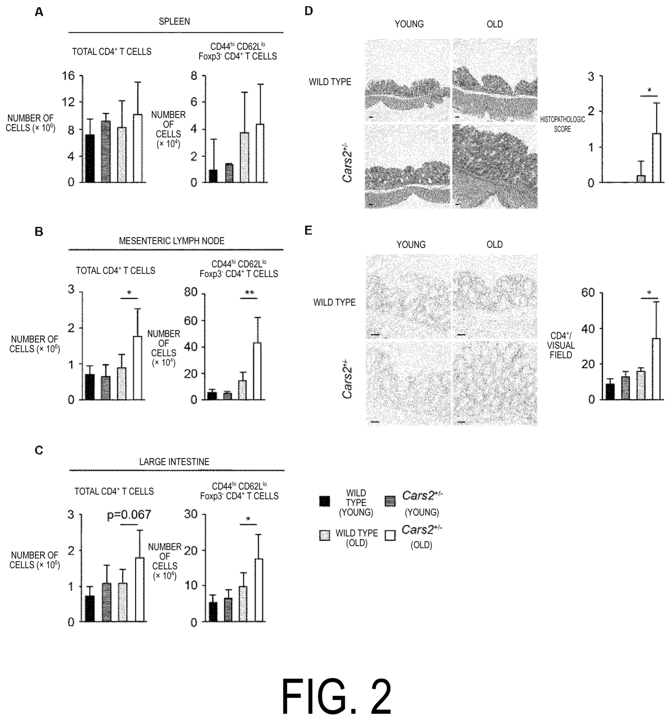

FIG. 2 shows that the number of effector/memory CD4+ T cells is enhanced in old Cars2+/- mice. (A to C) Numbers of total CD4+ T cells and CD44hi CD62Llo Foxp3- CD4+ T cells (mean ± standard deviation) accumulated in the (A) spleen, (B) mesenteric lymph nodes, and (C) large intestine of young (from 2 to 3 months) and old (12 months) wild-type or Cras2+/- mice (n = from 3 to 8). (D, E) Tissue images: (D) H & E staining and (E) CD4 immunostaining of the large intestine, and graphs: (D) histopathologic score and (E) number of CD4+ cells (mean ± standard deviation) (n = from 3 to 8). A scale bar indicates 50 µm. * and ** represent p < 0.05 and p < 0.01, respectively.

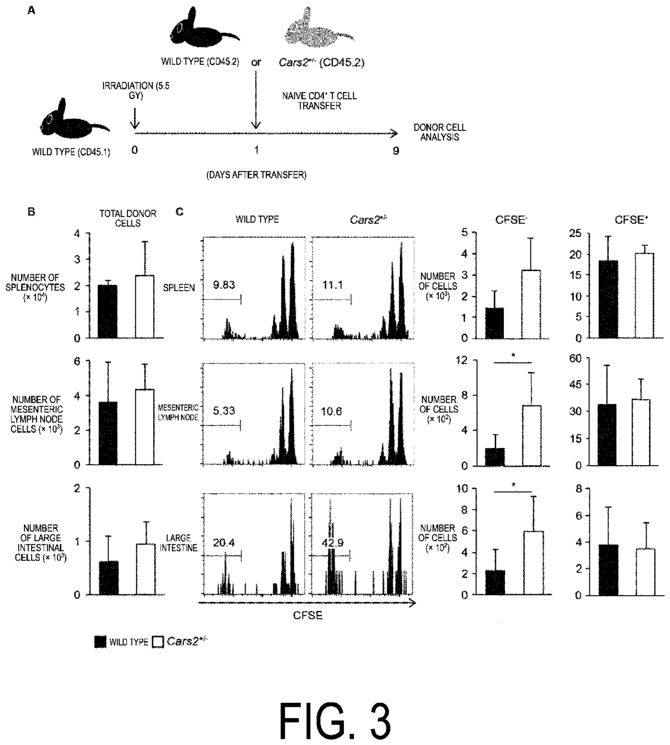

FIG. 3 shows excessive homeostatic maintenance proliferation of Cars2+/- CD4+ T cells during lymphopenia. (A) Experimental procedures. (B) Number of donor cells per organ (mean ± standard deviation) (n = 7 or 8). (C) Histogram: serial dilution of CFSE in donor cells accumulated in the respective organs, and graph: numbers of cells (mean ± standard deviation) in CFSE-negative (6 or more divisions) and CFSE-positive (0 to 2 divisions) populations in donor cells (n = 7 or 8). * represents p < 0.05.

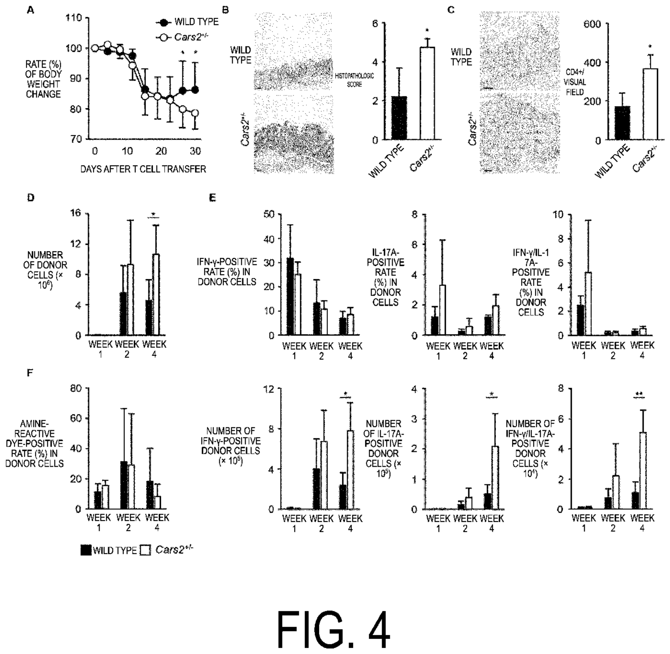

FIG. 4 shows that Cars2+/- CD4+ T cells exacerbate enteritis. (A) Rate of body weight loss (mean ± standard deviation) of host mice (n = 7 or 8). (B, C) Tissue images: (B) H & E staining and (C) CD4-positive cells; graphs: (B) histopathologic score (mean ± standard deviation) and (C) number of CD4-positive cells (mean ± standard deviation) (n = 4). (D) Graph: number of donor cells (mean ± standard deviation) accumulated in the large intestine at each time point after donor cell transfer (n = from 3 to 5). (E) (Top) frequency and (Bottom) absolute number (mean ± standard deviation) (n = from 3 to 5) of cytokine-producing cells. (F) Proportion of amine-reactive dye-positive cells (dead cells) (mean ± standard deviation) in donor cells accumulated in the large intestine (n = from 3 to 5). A scale bar indicates 50 µm. * and ** represent p < 0.05 and p < 0.01, respectively.

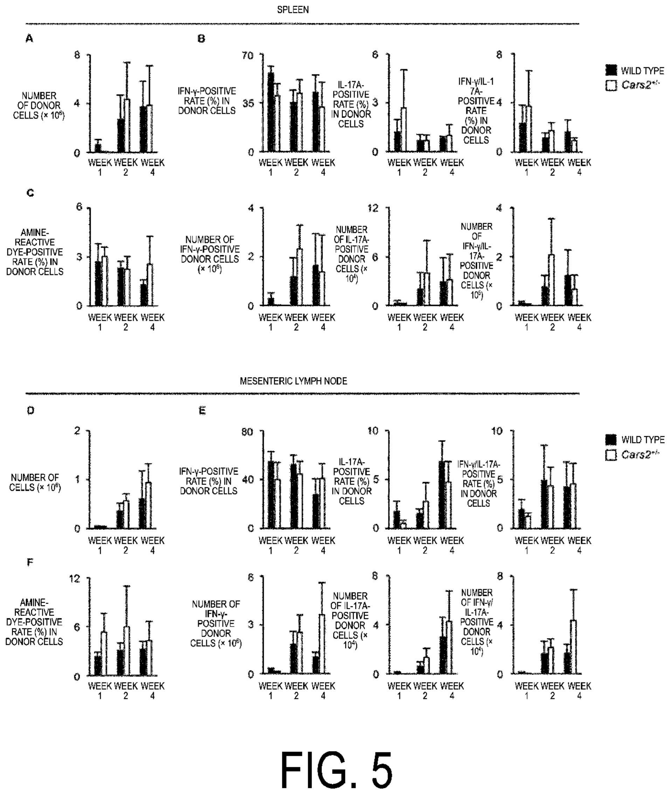

FIG. 5 shows that CARS2 does not affect the accumulation of CD4+ T lymphocytes in the spleen or mesenteric lymph nodes. (A) Number of donor cells (mean ± standard deviation) accumulated in the spleen at each time point after donor cell transfer (n = from 3 to 5). (B) (Top) frequency and (Bottom) absolute number (mean ± standard deviation) (n = from 3 to 5) of cytokine-producing cells in the spleen. (C) Proportion of amine-reactive dye-positive cells (dead cells) (mean ± standard deviation) in donor cells accumulated in the spleen (n = from 3 to 5). (D) Number of donor cells (mean ± standard deviation) accumulated in the mesenteric lymph nodes at each time point after donor cell transfer (n = from 3 to 5). (E) (Top) frequency and (Bottom) absolute number (mean ± standard deviation) (n = from 3 to 5) of cytokine-producing cells in the mesenteric lymph nodes. (F) Proportion of amine-reactive dye-positive cells (dead cells) (mean ± standard deviation) in donor cells accumulated in the mesenteric lymph nodes (n = from 3 to 5).

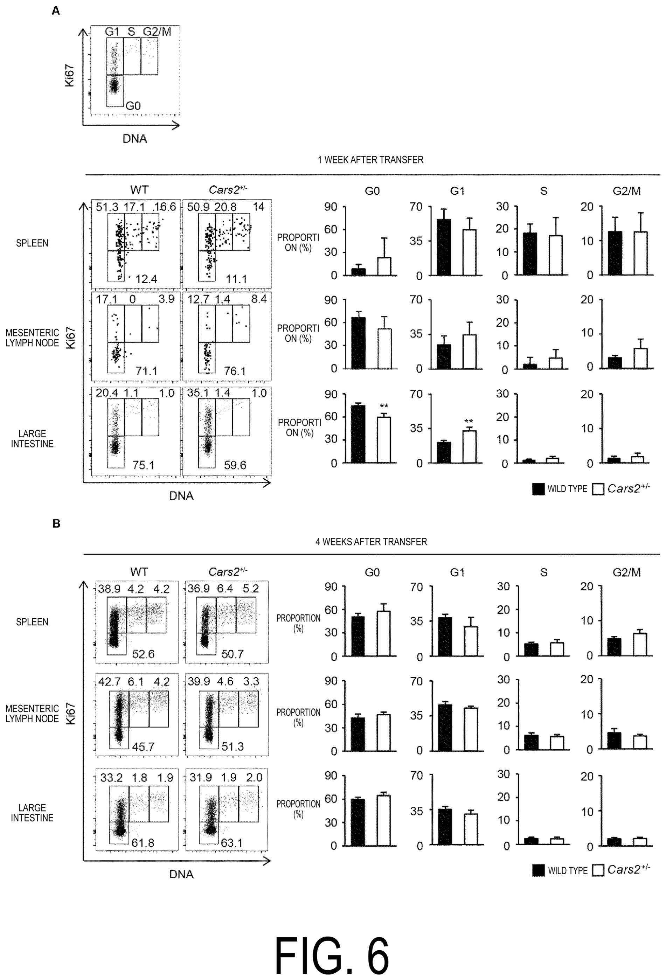

FIG. 6 shows that cell cycle entry is enhanced in Cars2+/- CD4+ T cells at an early stage of enteritis development. (A, B) Dot plots: expression level of Ki67 and total amount of DNA in donor cells, graphs: proportion of the same cells in each cell cycle (mean ± standard deviations) at (A) 1 week and (B) 4 weeks after donor cell transfer. ** represents p < 0.01.

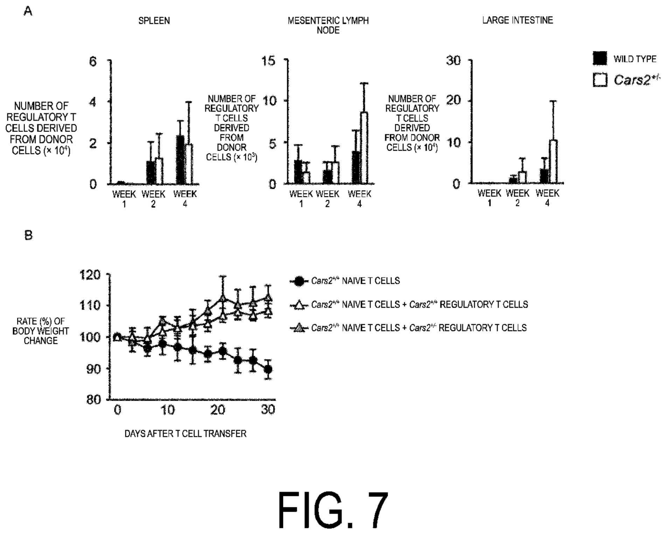

FIG. 7 shows that CARS2 attenuation does not affect the differentiation of regulatory T cells or their immunosuppressive capacity. (A) Number of newly differentiated regulatory T cells (mean ± standard deviation) at each time point after donor cell transfer. (B) Body weight variation ratio (mean ± standard deviation) (n = from 6 to 8).

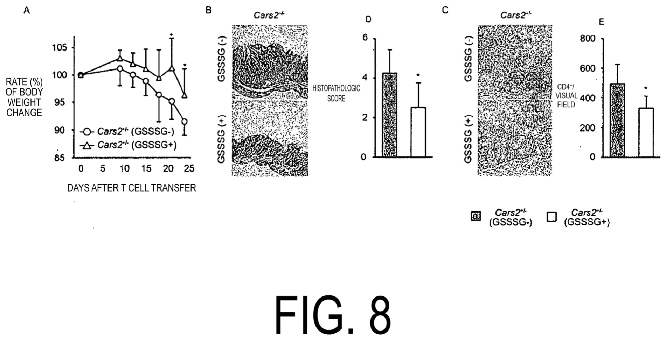

FIG. 8 shows that the enteritis induced by Cars2+/- CD4+ T cells is improved by administration of GSSSG. (A) Body weight variation (mean ± standard deviation) of host mice over time (n = from 6 to 8). (B, C) Tissue images: (B) H & E staining and (C) CD4 immunostaining are shown, and graphs: (D) histopathologic score and (E) number of CD4+ cells (mean ± standard deviation) (n = from 6 to 8). Scale bar: 50 µm. * represents p < 0.05.

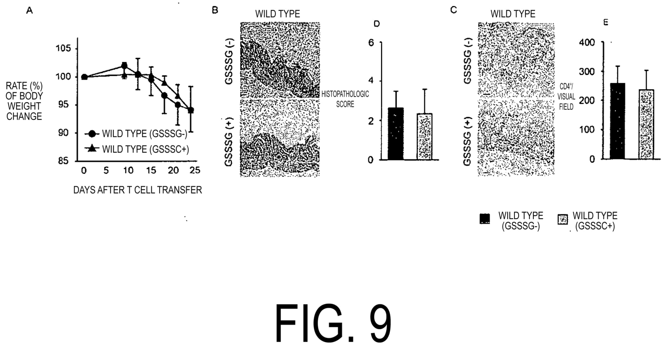

FIG. 9 shows results when GSSSG was administered to mice transfused with wild-type CD4+ T cells. (A) A graph shows body weight variation (mean ± standard deviation) of host mice over time (n = 7 or 8). (B, C) Tissue images: (B) H & E staining and (C) CD4 immunostaining, and graphs: (D) histopathologic score and (E) number of CD4-positive cells (mean ± standard deviation) (n = from 7 or 8). Scale bar: 50 µm. * represents p < 0.05.

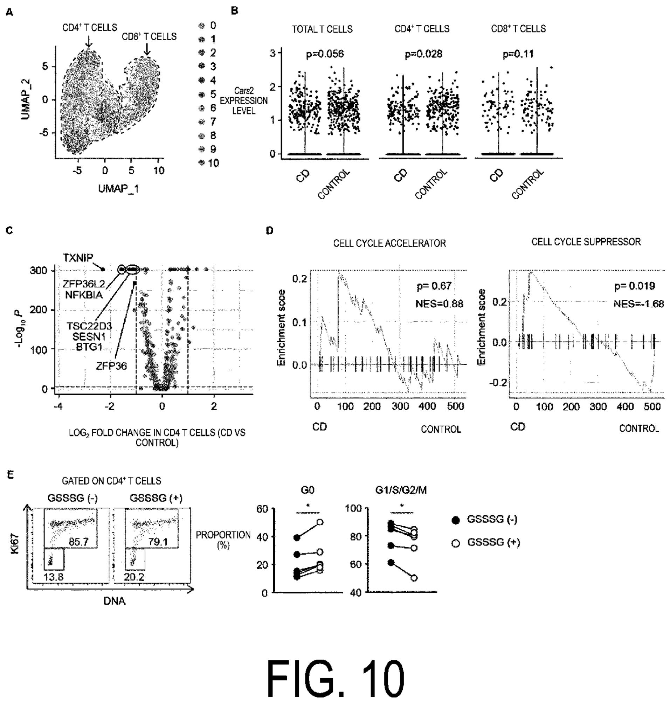

FIG. 10 shows that the regulation of the proliferation of CARS2-dependent CD4+ T cells is functional in humans. (A) Single-cell plot by a uniform manifold approximation and projection (UMAP) algorithm. (B) Violin plot showing expression levels of CARS2 in selected clusters. (C) Volcano plot showing genes whose expression was found to vary in CD4+ T cells derived from Crohn's disease as compared with controls. (D) GSEA analysis of factors that positively and negatively regulate the cell cycle in CD4+ T cells derived from Crohn's disease and controls. (E) Graph showing the proportion of human CD4+ T cells in each cell cycle (n = 6). * represents p < 0.05.

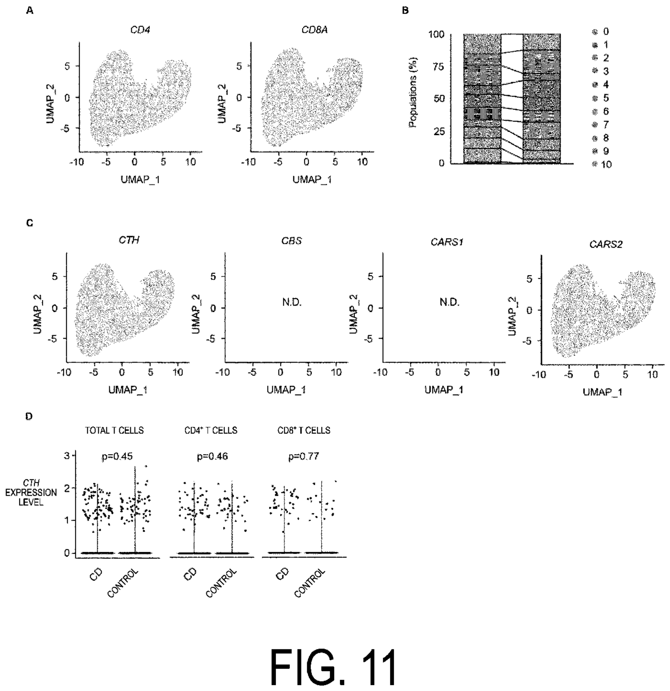

FIG. 11 shows subclustering of CD4+ T cells and CD8+ T cells of T lymphocytes derived from patients with human colitis using cellular RNA sequencing. (A, B) (A) Gene expression levels in CD4 and CD8A in the UMAP plot shown in FIG. 6A and (B) proportion of each cluster. (C) Expression levels of CTH, CBS, CARS1, and CARS2 in the UMAP plot shown in FIG. 6A. (D) A violin plot shows the expression level of CTH in intestinal tract CD4+ T cells. N. D. represents "not detected".

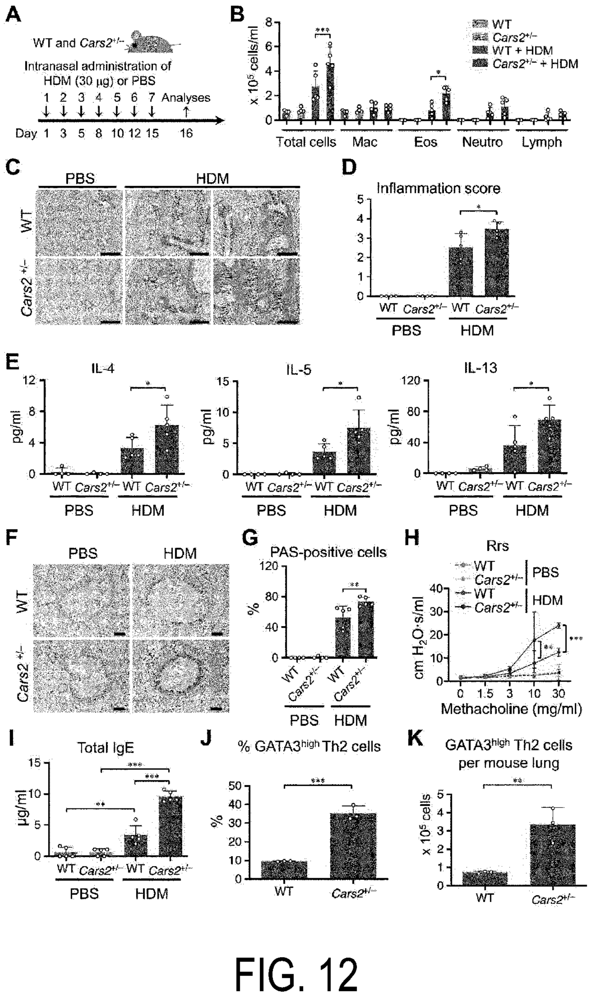

FIG. 12 shows enhancement of allergen-induced asthma in Cars2+/- mice. (A) Experimental protocol, (B) total number of cells and numbers of macrophages (Mac), eosinophils (Eos), neutrophils (Neutro), and lymphocytes (Lymph) in BALF. (C, D) (C) Photomicrographs of HE-stained lung tissue and (D) inflammation score. Scale bars: 500 µm for left 4 panels and 200 µm for right 2 panels. (E) Levels of type 2 cytokines IL-4, IL-5, and IL-13 in BALF. (F, G) (F) Photomicrographs of PAS-stained lung tissue and (G) number of PAS-positive cells in the airways. Scale bar: 50 µm (F). (H) Respiratory function measured by assessing bronchial hypersensitive reaction to methacholine. (I) Total serum IgE measured by ELISA. (J, K) Proportion of GATA3high Th2 cells in effector/memory CD3+ CD4+ FOXP3-CD44high T cells. Data are mean ± SD. P values were analyzed with one-way analysis of variance and Tukey's test. * represents p < 0.05; and ** represents p < 0.01. * represents p < 0.05; ** represents p < 0.01; and *** represents p < 0.001.

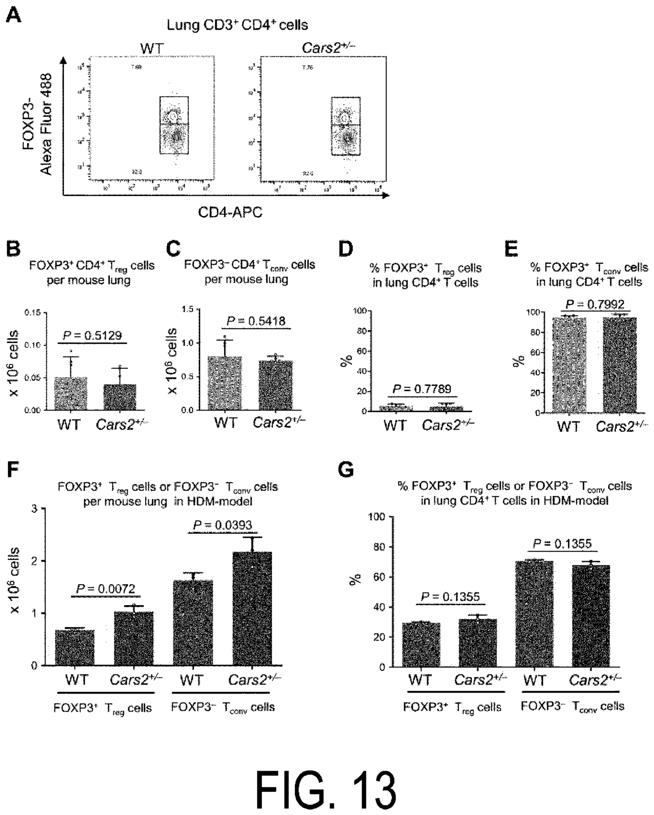

FIG. 13 shows FCM analysis of pulmonary regulatory T cells of wild-type and Cars2+/- mice. (A) FCM plots with antibody staining of FOXP3 and CD4 in pulmonary CD4+ T cells obtained from WT and Cars2+/- mice at steady state. (B to E), Numbers of (B) pulmonary regulatory T (Treg) cells and (C) typical CD4+ T (Tconv) cells, and proportions of (D) Treg cells and (E) Tconv cells in total pulmonary CD4+ T cells, in WT and Cars2+/- mice at steady state. (F, G) (F) Numbers of Treg cells and typical Tconv cells per mouse lung and (G) proportions of Treg cells and Tconv cells in total pulmonary CD4+ T cells, in HDM-treated WT and Cars2+/- mice.

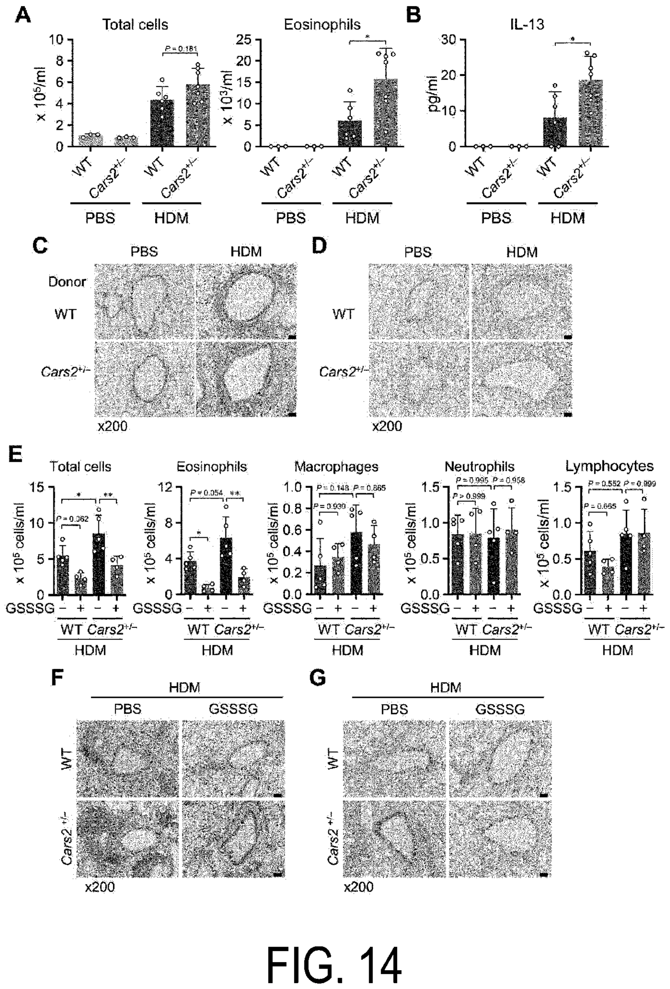

FIG. 14 shows contribution of CD4⁺ T cells to eosinophilic inflammation and therapeutic effects of GSSSG, in a mouse asthma model. (A, B) (A) Total number of cells and number of eosinophils in BALF of HDM- or PBS-treated Rag2-/- recipient mice after adoptive CD4+ T-cell transplantation and (B) IL-13 measurements obtained using ELISA. (C, D) Photomicrographs of (C) HE- and (D) PAS-stained mouse lung tissue. Scale bar: 50 µm. (E) Total number of cells and numbers of eosinophils, macrophages, neutrophils, and lymphocytes in BALF of HDM-treated mice (with/without GSSSG treatment). (F, G) Photomicrographs of (F) HE- and (G) PAS-stained mouse lung tissue. Scale bar: 50 µm. * represents p < 0.05; and ** represents p < 0.01.

### Description of Embodiments

The present invention relates to a pharmaceutical composition for treating or preventing an inflammatory bowel disease or a type 2 airway inflammation, containing a persulfide or a pharmaceutically acceptable salt or solvate thereof.

### 1. Active Sulfur Molecule

The active sulfur molecule is a compound having a polysulfide structure in which a plurality of sulfur atoms are added to a normal thiol group. The reduced hydropolysulfide compound has a significantly higher nucleophilicity of hydrosulfide itself than that of a normal thiol compound due to excessive addition of sulfur atoms. On the other hand, sulfur side chains present in the polysulfide structure also have nucleophilicity and thus exhibit various reactivities.

The "persulfide" used in the present invention is an active sulfur molecule, and is an oxidized persulfide represented by Formula (1) described below.

R¹S(S)ₙR² (1)

where R¹ and R² are independently selected from L-cysteine (Cys), N-acetylcysteine (NAC), homocysteine (Hcys), and glutathione (GSH), and represent moieties other than a thiol group; and n is an integer of 2 or more.

Glutathione is a tripeptide consisting of three amino acids, glutamic acid, cysteine, and glycine, and is denoted as GSH. GSSG in which two glutathione molecules are linked by a disulfide bond is called oxidized glutathione, and GSH is called reduced glutathione in some cases.

L-cysteine, N-acetylcysteine, homocysteine, and glutathione constituting the persulfide of the present invention may have a modified side chain as long as the object of the present invention is not impaired, and such derivatives are also included in the persulfide of the present invention.

In the present invention, n is not particularly limited as long as it is an integer of 2 or more, but n is preferably an integer of from 2 to 5, more preferably an integer of from 2 to 4, and still more preferably 2 or 3. Hereinafter, the persulfide represented by the above formula (1) is referred to as the persulfide of the present invention in some cases.

In the present invention, R¹ and/or R² is/are preferably glutathione, and both R¹ and R² are more preferably glutathione. A molecule in which an excessive sulfur atom is added to glutathione (oxidized type, reduced type) is called "glutathione persulfide", and examples thereof include GSSH, GSSSH, GSSG, GSSSG, and GSSSSG.

In the present invention, preferred glutathione persulfides include glutathione trisulfide (GSSSG), glutathione tetrasulfide (GSSSSG), glutathione pentasulfide (GSSSSSG) and the like. Among these, glutathione trisulfide or glutathione tetrasulfide is preferable, and glutathione trisulfide is particularly preferable.

The persulfide of the present invention may be in the form of a pharmaceutically (pharmacologically and pharmaceutically) acceptable salt (including ester) or solvate. Examples of the pharmaceutically acceptable salt include salts of inorganic acids or organic acids such as hydrochloric acid, hydrobromic acid, sulfuric acid, nitric acid, phosphoric acid, or citric acid, formic acid, fumaric acid, malic acid, acetic acid, succinic acid, tartaric acid, methanesulfonic acid, and p-toluenesulfonic acid; alkali metal or alkaline earth metal salts such as sodium, potassium, calcium, and magnesium; and basic amines or basic amino acid salts. The glutathione persulfide may be an ester with an alcohol or carboxylic acid having from 1 to 10 carbon atoms, preferably methyl alcohol, ethyl alcohol, acetic acid, or propionic acid. The pharmaceutically acceptable solvate includes hydrates.

The persulfide of the present invention is partially reduced in an environment (e.g., in vivo) to form RS(S)ₘH (m is an integer of 1 or more) as long as the object of the present invention is not impaired.

The persulfide of the present invention can be synthesized according to a previous report (Ida, T. et al. Proc. Natl. Acad. Sci. USA, 2014, doi: 10.1073/pnas). For example, N-acetylcysteine persulfide can be synthesized by reacting cysteine persulfide with sodium hydrogen sulfide in an aqueous medium (Zhang et al. Cell Chem. Biol, 2019, doi: 10.1016/j.chembiol. 2019.02.003). The glutathione persulfide can be synthesized by reacting reduced glutathione with an active sulfur donor (e.g., sodium sulfide, sodium hydrogen sulfide, another sodium sulfide such as sodium disulfide, trisulfide, or tetrasulfide, sodium thiosulfate, or an elemental sulfur such as S8) according to a well-known method. For example, the glutathione persulfide can be obtained by reacting reduced glutathione with sodium sulfide under argon conditions, stopping the reaction with formic acid and then centrifuging the reaction mixture, and subjecting the resulting supernatant to appropriate pre-treatment (e.g., Sep-Pak column) and HPLC (see WO2017/057768, supra).

### 2. Inflammatory Bowel Disease

One of diseases to be treated with the pharmaceutical composition of the present invention is an inflammatory bowel disease. The "inflammatory bowel disease" is a generic term for chronic diseases causing inflammations in the digestive tracts, and includes ulcerative colitis and Crohn's disease. The cause of the inflammatory bowel disease is unknown, but, unlike infectious bowel diseases, autoimmune mechanisms are believed to be involved in the disease. Ulcerative colitis is a nonspecific inflammatory disease of unknown cause that causes ulcers and erosions primarily in the large intestinal mucosa, with inflammation localized in the large intestine. Crohn's disease is an inflammatory disease of unknown cause that causes inflammation primarily in the entire digestive tract from the mouth to the anus.

The "inflammatory bowel disease" is, for example, a CD4⁺ T cell-mediated inflammatory bowel disease. The term "CD4⁺ T cell-mediated inflammatory bowel disease" means an inflammatory bowel disease induced by activation of CD4⁺ T cells or an inflammatory bowel disease accompanied by activation of CD4⁺ T cells.

The "inflammatory bowel disease" is, for example, an inflammatory bowel disease mediated by abnormal sulfur metabolism. The "inflammatory bowel disease mediated by abnormal sulfur metabolism " means an inflammatory bowel disease developed by abnormal sulfur metabolism or an inflammatory bowel disease developed in association with abnormal sulfur metabolism.

The "inflammatory bowel disease" is, for example, an inflammatory bowel disease induced by activation of CD4⁺ T cells due to abnormal sulfur metabolism, or an inflammatory bowel disease accompanied by activation of CD4⁺ T cells due to abnormal sulfur metabolism. The inflammatory bowel disease is preferably ulcerative colitis or Crohn's disease.

### 3. Type 2 Airway Inflammation

Another disease to be treated with the pharmaceutical composition of the present invention is "type 2 airway inflammation". The "type 2 airway inflammation" is a generic term for pathological conditions and symptoms accompanied by type 2 inflammations in the airways (an inflammatory response caused by Th2 cytokines as main factors through activation of Th2 cells and ICL2), and includes asthma (bronchial asthma), eosinophilic pneumonia, eosinophilic bronchiolitis, and allergic bronchopulmonary aspergillosis.

The "type 2 airway inflammation" is, for example, a CD4⁺ T cell-mediated type 2 airway inflammation. The term "CD4⁺ T cell-mediated type 2 airway inflammation" means a type 2 airway inflammation induced by activation of CD4⁺ T cells or a type 2 airway inflammation (such as asthma) accompanied by activation of CD4⁺ T cells.

The "type 2 airway inflammation" is, for example, a type 2 airway inflammation mediated by abnormal sulfur metabolism. The term "type 2 airway inflammation mediated by abnormal sulfur metabolism" means a type 2 airway inflammation developed by abnormal sulfur metabolism, or a type 2 airway inflammation (such as asthma) developed in association with abnormal sulfur metabolism.

The "type 2 airway inflammation" is, for example, a type 2 airway inflammation induced by activation of CD4⁺ T cells due to abnormal sulfur metabolism, or a type 2 airway inflammation (such as asthma) accompanied by activation of CD4⁺ T cells due to abnormal sulfur metabolism.

The "type 2 airway inflammation" is preferably asthma (bronchial asthma). Preferably, the "type 2 airway inflammation" is also not associated with an infection, and more preferably is non-viral.

### 4. Pharmaceutical Composition of Present Invention

The pharmaceutical composition of the present invention may contain the persulfide described above as an active ingredient, and may contain a pharmaceutically acceptable carrier or additive depending on the purpose of use or dosage form. Examples of such carriers and additives include, but are not limited to, excipients, binders, lubricants, solvents, disintegrants, solubilizers, suspending agents, emulsifiers, isotonizing agents, stabilizers, preservatives, antioxidants, flavoring agents, coloring agents, buffers, and fluidity promoters, and other commonly used carriers and additives can be used as appropriate.

Examples of the excipient include organic excipients such as sugars such as lactose, glucose, and D-mannitol, starches, and celluloses such as crystalline cellulose; and inorganic excipients such as calcium carbonate and kaolin.

Examples of the binder include pregelatinized starch, gelatin, gum arabic, methylcellulose, carboxymethylcellulose, sodium carboxymethylcellulose, crystalline cellulose, D-mannitol, trehalose, hydroxypropylcellulose, hydroxypropylmethylcellulose, polyvinylpyrrolidone, and polyvinyl alcohol.

Examples of the lubricant include fatty acid salts such as stearic acid and stearate, talc, and silicates.

Examples of the solvent include purified water, physiological saline, and a phosphate buffer.

Examples of the disintegrant include low-substituted hydroxypropyl cellulose, chemically modified cellulose, and starches.

Examples of the solubilizer include polyethylene glycol, propylene glycol, trehalose, benzyl benzoate, ethanol, sodium carbonate, sodium citrate, sodium salicylate, and sodium acetate.

Examples of the suspending agent or the emulsifier include sodium lauryl sulfate, gum arabic, gelatin, lecithin, glycerol monostearate, polyvinyl alcohol, polyvinylpyrrolidone, celluloses such as sodium carboxymethylcellulose, polysorbates, and polyoxyethylene hydrogenated castor oil.

Examples of the isotonizing agent include sodium chloride, potassium chloride, sugars, glycerin, and urea.

Examples of the stabilizer include polyethylene glycol, dextran sulfate sodium, other amino acids, and magnesium carbonate which is also an acidity regulator.

Examples of the preservative include para-hydroxybenzoic acid esters, chlorobutanol, benzyl alcohol, phenethyl alcohol, dehydroacetic acid, and sorbic acid.

Examples of the antioxidant include water-soluble antioxidants such as ascorbic acid, cysteine hydrochloride, sodium bisulfate, sodium metabisulfite, and sodium sulfite, fat-soluble antioxidants such as ascorbyl palmitate, butylated hydroxyanisole (BHA), butylated hydroxytoluene (BHT), lecithin, propyl gallate, and α-tocopherol, and metal chelating agents such as citric acid, ethylenediaminetetraacetic acid (EDTA), sorbitol, tartaric acid, and phosphoric acid.

Examples of corrigents include sweeteners and flavors that are commonly used in the pharmaceutical field, and examples of the coloring agent include colorants that are commonly used in the pharmaceutical field.

The pharmaceutical composition of the present invention can be formulated into pharmaceutical preparations such as tablets (including sugar-coated tablets, film-coated tablets, sublingual tablets, and orally disintegrating tablets), powders, granules, capsules (including soft capsules and microcapsules), liquids, troches, syrups, emulsions, suspensions, injections (e.g., subcutaneous injections, intramuscular injections, and intraperitoneal injections), external preparations (e.g., transnasal administration preparations, transdermal preparations, and ointments), suppositories (e.g., rectal suppositories and vaginal suppositories), foams (enema), pellets, transnasal preparations, and transpulmonary preparations (inhalants).

The pharmaceutical composition of the present invention is administered to a subject in a therapeutically effective amount. The "therapeutically effective amount" refers to an amount of a therapeutic agent that is useful to alleviate a selected condition. The therapeutically effective amount is appropriately determined depending on the purpose of use, the route of administration, the age and symptoms of the patient, and the like. In general, the persulfide is administered to the subject orally at a dose of from 0.1 to 1000 mg/kg of body weight, preferably about from 0.1 to 100 mg/kg of body weight, or parenterally (e.g., by injections or drip infusions) at a dose of from 0.01 to 100 mg/kg of body weight, preferably about from 0.1 to 10 mg/kg of body weight. One type of persulfide or two or more types thereof may be used.

The route of administration of the pharmaceutical composition of the present invention is not particularly limited, and may be oral or parenteral. Specific examples of the parenteral administration include injection administration, transnasal administration, transpulmonary administration, and transdermal administration. Examples of the injection administration include intravenous injection, intramuscular injection, intraperitoneal injection, subcutaneous injection, intramedullary injection, intrathecal injection, and intradermal injection. The administration method can be appropriately selected depending on the age and symptoms of the subject.

In the pharmaceutical composition of the present invention, the persulfide as an active ingredient can prevent and treat an inflammatory bowel disease or a type 2 airway inflammation by regulating (suppressing) abnormal activation or proliferation of CD4⁺ T cells in the inflammatory bowel disease or the type 2 airway inflammation.

### 5. CD4⁺ T cell modulator

The present invention provides a CD4⁺ T cell modulator, containing a persulfide or a pharmaceutically acceptable salt or solvate thereof as an active ingredient. The persulfide as an active ingredient is as described in the above 1.

The CD4⁺ T cell modulator means an agent that regulates (suppresses) abnormal activation or proliferation of CD4⁺ T cells, and enables prevention or treatment of symptoms or diseases accompanied by abnormal activation or proliferation of CD4⁺ T cells, including inflammatory bowel diseases and type 2 airway inflammations.

### Examples

Hereinafter, the present invention will be described specifically with reference to examples, but the present invention is not limited to these examples.

### [Example 1] Effect of Glutathione Persulfide on Inflammatory Bowel Disease Methods and Materials:

### Mice:

C57BL/6 CD45.2⁺ mice were purchased from Japan SLC, Inc. Rag2^{-/-} mice and CD45.1⁺ congenic mice were obtained from the breeding stock of Graduate School of Medicine, Tohoku University. Foxp3-RFP reporter mice were purchased from Jackson Laboratory. Cars2^{+/-} mice used were created according to a previous report (Akaike T et al, Nat Commun 8: 1177, 2017, DOI: 10.1038/s41467-017-01311-y). Cars2^{+/-}Foxp3-RFP reporter mice were obtained by crossing Foxp3-RFP reporter mice with Cars2^{+/-} mice.

### Oxidized Glutathione Trisulfide (GSSSG):

GSSSG was synthesized according to previous reports (Ida et al., Proc Natl Acad Sci U S A 111, 7606-7611 (2014), Akaike et al., Nat Commun 8, 1177 (2017)., and Takata et al., Nitric Oxide 116, 47-64 (2021)).

### Cell Transfer:

CD4⁺ T cells were magnetically sorted from splenocytes of young mice (from 8 to 12 weeks old). Then, naive CD4⁺ T cells (CD3⁺ CD4⁺ CD25⁻ CD44^{lo} CD62L^{hi} or CD3⁺ CD4⁺ Foxp3-RFP⁻ CD44^{lo} CD62L^{hi}) were sorted using FACS AriaII (BD Bioscience) based on fluorescently labeled monoclonal antibodies or reporter fluorescent proteins. In experiments to confirm homeostatic maintenance proliferation, naive CD4⁺ T cells were labeled with carboxyfluorescein succinimidyl ester (CFSE) (Thermo Fisher Scientific), and were injected into the tail veins of CD45.1 host mice irradiated with a sublethal dose (5.5 Gy), at 1 × 10⁶ cells per mouse 1 day before transfer. In experiments to induce enteritis, wild-type or Cars2^{+/-} naive CD4⁺ T cells were injected into the tail veins of Rag2^{-/-} mice at 3 × 10⁵ cells per mouse. In experiments to evaluate the function of regulatory T cells, Cars2^{+/+} naive CD4⁺ T cells were co-transferred with regulatory T cells derived from Cars2^{+/-} Foxp3-RFP reporter mice (1 × 10⁵ cells/mouse). In GSSSG administration experiments, GSSSG (400 nmol/mouse) was intraperitoneally administered to the Rag2^{-/-} host mice every day from the same day as the day when the naive CD4⁺ T cells were transferred.

### Histological Evaluation:

At the stage where from 3 to 4 weeks had elapsed after the transfer of the naive T cells, the large intestines of the Rag2^{-/-} host mice were collected and fixed with 10% formalin. After the fixed samples were embedded in paraffin, 5-µm-thick sections were cut out and subjected to hematoxylin-eosin staining (H & E) and immunostaining with a CD4 monoclonal antibody (mAb) (EPR19514). The histological inflammation score was classified into three groups with reference to a previous report (Biswas et al., Nat Commun 9, 1779 (2018)).

### Lymphocyte Isolation:

The thymus, spleen and mesenteric lymph nodes were crushed on a 40 µm cell strainer (greiner bio one) to obtain a cell suspension. In the cell extraction from the spleen, red blood cell hemolysis treatment was further performed with a hemolytic buffer. Purification of a cell suspension from the intestinal lamina propria was performed using the Lamina Propria dissociation kit (Miltenyi Biotec) according to the manufacturer's instructions. Lymphocytes were then isolated using Percoll (GE Healthcare).

### Flow Cytometry Analysis

Flow cytometry was performed using LSR Fortessa (BD Bioscience). Data analysis was performed using Flowjo (BD Bioscience) software.

### Isolation and Culture of Human Naive CD4⁺ T Cells

Peripheral mononuclear blood cells were isolated using Vacutainer (BD Bioscience), and naive CD4⁺ T cells (CD3⁺ CD4⁺ CD25⁻ CD45RO⁻ CD45RA⁺) were purified from the isolated mononuclear cells using FACS AriaII (BD Bioscience). The purified naive CD4⁺ T cells were seeded in a 96-well plate at 5 × 10⁴ cells per well, and stimulated with Dynabeads^{™} Human T-Activator CD3/CD28 (Thermo Fischer Scientific) in an RPMI medium (10% FCS) in the presence or absence of GSSSG (1 µM) for 48 hours.

### Results:

### 1. Enhanced Accumulation of Effector/Memory CD4+ T Cells in Large Intestine of Old Cars2+/- Mice

To confirm whether CARS2-dependent active sulfur metabolism is functional in CD4+ T cells, various thymic T cell fractions were compared between young (from 2 to 3 months) and old (12 months) wild-type and Cars2+/- mice. As a result, no significant change was observed in each thymic T cell fraction in the Cars2+/- mice as compared with the wild-type mice (A and B of FIG. 1), revealing that the CARS2-dependent sulfur metabolism does not affect the generation of T cells.

Next, to assess the influence of CARS2 on the homeostatic maintenance of CD4+ T cells in the periphery, the properties of CD4+ T cells in the spleen, mesenteric lymph nodes and large intestine were compared between young and old wild-type and Cars2+/- mice. While there were no significant differences between the two mice in the various fractions of CD4+ T cells in the spleen, there were increases in numbers of CD4+ T cells accumulated in the mesenteric lymph nodes and large intestine tissue, and particularly the CD44hi CD62Llo effector/memory subset in the old Cars2+/- mice (A to C of FIG. 2). Furthermore, the infiltration of CD4⁺ T lymphocytes was histologically increased in the large intestines of the old Cars2+/- mice, and accordingly, the enhancement of inflammatory infiltration and hyperplasia of epithelial cells were confirmed in the same tissue (D and E of FIG. 2). The above results showed that CARS2 suppressed the accumulation of effector/memory CD4+ T cells in the large intestine and gut-associated lymphoid tissues.

### 2. Suppression of Homeostatic Maintenance Proliferation of Intestinal Tract CD4+ T Cells by CARS2

The numbers of naive T cells and effector/memory T cells in the periphery are maintained by a proliferative response called "homeostatic maintenance proliferation". This homeostatic maintenance proliferation can be experimentally induced by making mice lymphopenic, and it is known that when naive CD4+ T cells are transferred into genetically modified mice or sublethally irradiated lymphopenic mice, the transferred T cells rapidly proliferate in lymphoid and non-lymphoid tissues to produce effector/memory T cells. Therefore, in order to confirm whether the increases in numbers of effector/memory T cells accumulated in the mesenteric lymph nodes and large intestine observed in the old Cars2+/- mice (FIG. 2) are due to enhanced homeostatic maintenance proliferation, naive CD4+/- cells derived from wild-type and Cars2+ T mice were transferred to lymphopenic mice, and compared in terms of the degree of homeostatic maintenance proliferation.

Specifically, naive T cells sorted from wild-type or Cars2+/- mice were CFSE-labeled and then transferred into sublethally irradiated lymphopenic wild-type mice, and after 9 days, donor cells accumulated in the spleen, mesenteric lymph nodes, and large intestine were analyzed (experimental procedures: A of FIG. 3). As a result, the same number of total donor cells and cell populations with a low proliferation rate (CFSE-positive) were observed between the wild-type and Cars2+/- (B and C of FIG. 3), but populations with a high proliferation rate (CFSE-negative) were significantly increased in the Cars2+/- CD4+ T cells (C of FIG. 3). These results showed that CD4+ T cell-endogenous CARS2 acts to suppress rapid homeostatic maintenance proliferation of the same cells.

### 3. Suppression of Proliferation of Enteritis-Induced CD4+ T Cells by CARS2

The rapid proliferation of Cars2+/- CD4+ T cells in the large intestine as compared with the wild type suggested that the Cars2+/- T cells may exacerbate enteritis under certain circumstances. When naive CD4+ T cells are transferred into lymphopenic mice (Rag2-/- mice), the transferred naive CD4+ T cells exhibit rapid homeostatic maintenance proliferation, and Crohn's disease-like IBD is induced. Therefore, using the same IBD model, wild-type and Cars2+/- naive CD4+ T cells were adoptively transferred to Rag2-/- mice, and the properties of the donor cells and the IBD pathological conditions of the host mice were observed over time. The Rag2-/- host mice that received adoptive transfer of Cars2+/- CD4+ T cells showed more severe body weight loss and histological exacerbations of colitis as compared with the wild-type transfer group (FIGS. 4A to 4C). In addition, when the numbers of donor T cells accumulated in the respective organs were compared, no difference was observed in the lymphoid tissue (FIGS. 5A and 5D), but the number of Cars2+/- donor cells accumulated in the large intestine was larger than that in the wild type (FIG. 4D). At Week 4 of transfer, no difference was observed in the proportions of effector cytokine-producing cells (IFN-γ, IL-17A, IFN-γ/IL-17A) in the donor T cells accumulated in the respective organs, but the absolute number of Cars2+/- effector cytokine-producing T cells increased in proportion to the increase in number of Cars2+/- donor cells (FIGS. 4E, 5B, and 5E). Since there was no significant difference in proportion of dead cells between the two groups (FIGS. 4F, 5C, and 5F), the reason for the increase in number of accumulated cytokine-producing Cars2+/- T cells is considered to be enhanced homeostatic maintenance proliferation of the donor cells.

To verify the above hypothesis, the cell cycle of the donor cells in the large intestine tissue was analyzed. 1 week after transfer of naive CD4+ T cells, the proportion of the donor cells in each cell cycle was compared, and the proportion in the G0 phase was reduced, while the proportion in the G1 phase was enhanced for the Cars2+/- T cells, as compared with that for the wild type, and there was no difference in proportions in the S and G2/M phases (FIG. 6A). The differences observed in the G0 and G1 stages were observed only in the large intestine, and was not observed in the other lymphoid tissues (FIG. 6A), and no difference was observed in any organs 4 weeks after the transfer of T cells (FIG. 6B). The above results showed that the pathogenic CD4+ T cell-endogenous CARS2 suppressed the cell cycle entry of T cells at the initial stage of the inflammatory response.

### 4. Differentiation and Function of Cars2+/- Regulatory T Cells

It is known that some of naive CD4+ T cells transferred into lymphopenic mice differentiate into Foxp3+ regulatory T cells having an immune response suppressing function. Therefore, it was examined whether CARS2 affects the differentiation and immunosuppressive capacity of regulatory T cells. Specifically, the number of Foxp3+ T cells derived from donor cells transferred into Rag2-/- mice was observed over time to perform evaluation. As shown in FIG. 7A, no difference was observed in number of regulatory T cells differentiated from naive T cells, and it was found that CARS2 is not essential for the differentiation of regulatory T cells. In addition, to confirm whether CARS2 affects the immunosuppressive function of regulatory T cells, Cars2+/+ or Cars2+/- regulatory T cells were co-infused with Cars2+/+ Foxp3- naive CD4+ T cells. As a result, it was revealed that these two types of regulatory T cells have equivalent enteritis suppressing capacity (FIG. 7B). This suggests that CARS2 may not significantly affect the differentiation and function of regulatory T cells.

### 5. Suppression of Enteritis Induced by Cars2+/- CD4+ T Cells by GSSSG

To confirm whether CARS2 suppresses the proliferation of enteritis pathogenic T cells via active sulfur, GSSSG, which is a donor of an active sulfur molecule, was administered to Rag2-/- host mice, and as a result, the body weight loss of the individuals transplanted with Cars2+/- CD4+ T cells was suppressed, and the reduction in histological enteritis images was observed (FIGS. 8A to 8C). On the other hand, no significant therapeutic effect was observed in the individuals transfused with the wild-type CD4+ T cells (FIGS. 9A to 9C). These results suggested that the exacerbation of enteritis induced by the Cars2+/- CD4+ T cells was due to a decrease in number of active sulfur molecule species in the same cells.

### 6. Correlations between Reduced Expression of CARS2 in Human CD4+ T Cells and Development of IBD

The results so far showed that the reduction in CARS2-dependent active sulfur metabolism in mice resulted in excessive proliferation of CD4+ T lymphocytes and exacerbation of enteritis. Therefore, in order to examine whether the reduced expression of CARS2 is associated with the development of human IBD, the public database (GSE15477), in which single-cell RNA analysis of T cells collected from the large intestines of patients with Crohn's disease was performed, was reanalyzed. By cluster analysis of T lymphocytes, they were classified into 11 subclusters belonging to CD4+ (clusters 1, 3 to 6, 8, and 9) and CD8+ (clusters 0, 2, 7, and 10) (FIGS. 10A, 11A, and 11B). Interestingly, the expression of CARS2 was reduced in the CD4+ T cells of patients with Crohn's disease, but no difference was observed in expression of the CD8+ T cells (FIGS. 10B and 14C). This suggested that reduced expression of CARS2 in human CD4+ T cells is associated with the development of IBD. On the other hand, no difference was observed in expression level of CTH involved in the production of active sulfur between the two groups (FIGS. 11C and 11D). Furthermore, genes whose expression varied between patients with Crohn's disease in CD4+ T cells in the large intestine tissue and controls were identified using the same database. As a result, the expression of genes that suppress the activation and proliferation of T cells (TXNIP, ZFP36L2, BTG1, SESN, ZFP36, and NFKBIA) was reduced in the CD4+ T cells of patients with Crohn's disease (27-32) (FIG. 10C), and a group of genes that negatively regulate cell proliferation was significantly attenuated in patients with Crohn's disease (FIG. 10D). On the other hand, no variation was observed in factors that positively regulate proliferation (FIG. 10D). Therefore, it was suggested that the proliferation of the CD4+ T cells infiltrated into the large intestine tissue of patients with Crohn's disease may be enhanced. These results suggested that the proliferation of CD4+ T cells is enhanced by the reduced expression of CARS2-dependent active sulfur metabolism in humans, which may lead to the exacerbation of IBD.

Finally, the involvement of active sulfur in the regulation of the proliferation of human CD4+ T cells was examined through in vitro cell culture. Specifically, naive CD4+ T cells purified from peripheral bloods of healthy individuals were stimulated with CD3/CD28 in the presence and absence of GSSSG to induce the proliferative response, and the cell cycle was analyzed 48 hours after the stimulation. As a result, the addition of GSSSG significantly suppressed the cell cycle entry of the naive CD4+ T cells (FIG. 10E). Thus, it was shown that CARS2-dependent active sulfur metabolism also suppresses the cell cycle entry also in human CD4+ T cells and limits the proliferation of the same cells.

### [Example 2] Effect of Glutathione Persulfide on Type 2 Airway Inflammation Methods and Materials:

### Type 2 Airway Inflammation Asthma (HDM-Induced Mouse Asthma) Model

Asthma model mice were created according to a previous report (Ishii et al., Scandinavian journal of immunology. 2018; 87 (3)). Specifically, as shown in the FIG. 5A, mice were intranasally administered with HDM extract (at the indicated doses in PBS) under isoflurane anesthesia to induce asthma.

### BALF Cell Analysis

Twenty four (24) hours after the last dose of HDM, the mice were anesthetized and sacrificed by exsanguination. The alveoli were washed three times with 500 µL of 10 mM PBS, and the bronchial alveolar lavage fluid (BALF) was collected. For 5 minutes, 800 g of BALF was centrifuged, then the supernatant was stored for biochemical analysis, and the cellular pellet was resuspended in 500 µL of 10 mM PBS. The total number of cells was counted using a Scepter handheld automated cell counter (Millipore), followed by cytospin specimen preparation and staining with Diff-Quick (Sysmex) or Eosinostain-Torii (Torii Pharmaceutical).

### Isolation of Cells from Mouse Lung Tissue and FCM, and Morphological Analysis of Lung Section

Isolation of cells from mouse lung tissue and FCM were performed according to previous reports (Gomez et al., American journal of respiratory cell and molecular biology. 2015; 52 (3): 349-64., and Yamada et al., American journal of respiratory and critical care medicine. 2011; 183 (10): 1391-401.). For histological examination of lung inflammation, lung sections were stained with hematoxylin and eosin (HE). Lung inflammation was semi-quantified and evaluated according to a previous report.

### Measurement of Respiratory Function in Mice

The mouse respiratory function was assessed by measuring airway resistance 24 hours after the last dose of the HDM extract using the flexiVent system (SCIREQ).

### Results:

### 1. Severe Type 2 Airway Inflammation Asthma In Cars2/- Mice

To verify the effect of CARS2 blockade on TCR signaling, the present inventors subjected CARS2/- mice to intranasal administration of HDM extract which is an allergen in mouse models of asthma that induces a type 2 airway inflammation (FIG. 12A). No significant differences were observed in total number of cells or cell difference in bronchial alveolar lavage fluid (BALF) occurred between phosphate-buffered saline (PBS)-treated Cars2+/- and WT mice (FIG. 12B). However, the Cars2+/- mice showed increased eosinophilic inflammation in the airways and lungs after HDM treatment as compared with the WT mice (FIGS. 12B to 12D). The HDM-treated Cars2+/- mice had enhanced release of type 2 cytokines including IL-4, IL-5 and IL-13 in the airways as compared with the HDM-treated WT mice (FIG. 12E). Alongside the enhanced type 2 inflammatory response in the HDM-treated Cars2+/- mice, goblet cell hyperplasia in the airways of the HDM-treated Cars2+/- mice was increased as compared with that in the airways of the HDM-treated WT mice (FIGS. 12F and 12G). Bronchial hyperresponsiveness (Rrs, resistance of the respiratory system) was also enhanced in the HDM-treated Cars2+/- mice (FIG. 12H). In addition, the serum IgE level was significantly increased in the HDM-treated Cars2+/- mice as compared with that in the WT mice (FIG. 12I). In the lungs of the HDM-treated CARS2+/- mice, the number of GATA3high Th2 cells was significantly increased (FIGS. 12J and 12K), suggesting that CARS2 heterozygous disruption enhances an allergen-induced type 2 airway inflammation via activation of naive CD4 T cells and enhanced Th2 differentiation.

Both the number of regulatory T cells (Tregs) and the proportion of Tregs in the total pulmonary CD4 T cells in the Cars2+/- mice at steady state were not significantly different from those in the WT mice (FIGS. 13A to 13E). The number of total pulmonary CD4 T cells, Tregs and typical FOXP3-negative T cells in the lungs of the HDM-treated Cars2+/- mice was higher than that in the WT mice (FIG. 13F). However, the percentage of Tregs in the total pulmonary CD4 cells in the Cars2+/- mice was not different from that in the WT mice (FIG. 13G). These data suggest that the enhanced type 2 immune responses in the HDM-treated Cars2+/- mice are not due to the difference in Treg cells between the WT mice and the Cars2+/- mice.

### 2. Type 2 Airway Inflammation by T Cell CARS2 Blockade

To confirm whether CARS2 blockade in CD4 T cells is responsible for the exacerbation of a type 2 airway inflammation in this model, purified CD4 T cells derived from CARS2+/- or WT mice were then transferred into Rag2-/- mice and the recipient mice were treated with HDM or PBS. The HDM-treated Rag2-/- mice reconstituted with Cars2+/- CD4 T cells showed increased accumulation of eosinophils in the airways (FIG. 14A) and enhanced release of the type 2 cytokine IL-13 (FIG. 14B). Consistent with these findings, lung inflammation (FIG. 14C) and goblet cell hyperplasia (FIG. 14D) were also enhanced in the HDM-treated Rag2-/- mice reconstituted with Cars2+/- CD4 T cells as compared with the mice reconstituted with WT CD4 T cells. These data demonstrate that CARS2 blockade increased type 2 responses via a T cell-mediated mechanism consistent with the present inventors' in vitro observations of enhanced TCR signaling in the CARS2+/- CD4 T cells.

### 3. Alleviation of Type 2 Airway Inflammation by GSSSG

It was investigated whether intraperitoneal administration of GSSSG in vivo suppresses an HDM-induced type 2 airway inflammation. The treatment with 1 mM GSSSG significantly suppressed the accumulation of inflammatory cells, particularly eosinophils, in the airways of both the Cars2+/- mice and the WT mice (FIG. 14E). Furthermore, the GSSSG treatment significantly reduced lung inflammation (FIG. 14F) and goblet cell hyperplasia (FIG. 14G) in both the HDM-treated Cars2+/- mice and the HDM-treated WT mice. Thus, GSSSG has therapeutic potential in CD4 T cell-mediated type 2 inflammatory diseases, including asthma, by organizing TCR signaling.

In the above examples, GSSSG, which is GS(S)nG, was used. However, even when CysS(S)nCys, NACS(S)nNAC, or HcysS(S)nHcys is administered instead of GS(S)nG, the administered CysS(S)nCys, NACS(S)nNAC, or HcysS(S)nHcys is metabolized and converted into GS(S)nG in vivo (See, Ida et al. and Zhang et al., supra.). Therefore, CysS(S)nCys, NACS(S)nNAC, and HcysS(S)nHcys are also expected to exhibit the same effects as GS(S)nG disclosed herein.

### Industrial Applicability

The present invention is useful in the prevention and treatment of CD4 T cell-mediated diseases such as inflammatory bowel diseases and type 2 airway inflammations including asthma.

All publications, patents and patent applications cited herein are hereby incorporated by reference in their entirety.

## Claims

1. A pharmaceutical composition for treating or preventing an inflammatory bowel disease or a type 2 airway inflammation, the pharmaceutical composition comprising a persulfide represented by Formula (1) described below or a pharmaceutically acceptable salt or solvate thereof:
R¹S(S)ₙR² (1),
wherein R¹ and R² are independently selected from L-cysteine, N-acetylcysteine, homocysteine, and glutathione (GSH), and represent moieties other than a thiol group; and n is an integer of 2 or more.

2. The pharmaceutical composition according to claim 1, wherein R¹ and R² are moieties other than a thiol group of glutathione.

3. The pharmaceutical composition according to claim 1, wherein the persulfide is glutathione trisulfide or glutathione tetrasulfide.

4. The pharmaceutical composition according to claim 1, wherein the inflammatory bowel disease is Crohn's disease or ulcerative colitis.

5. The pharmaceutical composition according to claim 1, wherein the type 2 airway inflammation is asthma.

6. The pharmaceutical composition according to any one of claims 1 to 5, wherein the persulfide treats or prevents the inflammatory bowel disease or the type 2 airway inflammation by regulating activation or proliferation of CD4⁺ T cells.

7. A CD4⁺ T cell modulator comprising, as an active ingredient, a persulfide represented by Formula (1) described below or a pharmaceutically acceptable salt or solvate thereof:
R¹S(S)ₙR² (1),
wherein R¹ and R² are independently selected from L-cysteine, N-acetylcysteine, homocysteine, and glutathione (GSH), and represent moieties other than a thiol group; and n is an integer of 2 or more.

8. The CD4⁺ T cell modulator according to claim 7, wherein R¹ and R² are moieties other than a thiol group of glutathione.

9. The CD4⁺ T cell modulator according to claim 7 or 8, wherein the persulfide is glutathione trisulfide or glutathione tetrasulfide, preferably glutathione trisulfide.
